Europäisches Patentamt

(19) European Patent Office

Office européen des brevets

(11) Publication number: **0 346 297**
A1

# EUROPEAN PATENT APPLICATION

(21) Application number: **89830239.3**

(22) Date of filing: **30.05.89**

(51) Int. Cl.⁴: **A 61 K 31/485**
A 61 K 9/06
//(A61K31/485,31:415,31:21)

(30) Priority: **08.06.88 IT 6753488**

(43) Date of publication of application:
**13.12.89 Bulletin 89/50**

(84) Designated Contracting States:
**AT BE CH DE ES FR GB GR LI NL SE**

(71) Applicant: **CENTRO MEDICO HARVEY S.r.l.**
**Corso Vittorio Emanuele II, 86**
**I-10121 Torino (IT)**

(72) Inventor: **Pastorini, Silvio**
**Corso Unione Sovietica 324**
**I-10135 Torino (IT)**

(74) Representative: **Rambelli, Paolo et al**
**Jacobacci-Casetta & Perani S.p.A. Via Alfieri 17**
**I-10121 Torino (IT)**

(54) A preparation for topical use in the therapeutic treatment of impotentia coeundi.

(57) A preparation for topical use in the therapeutic treatment of impotentia coeundi includes papaverine, phentolamine and possibly nitroglycerine as active agents in a vehicle preferably constituted by a gel.

EP 0 346 297 A1

Bundesdruckerei Berlin

**Description**

**A preparation for topical use in the therapeutic treatment of impotentia coeundi**

The present invention relates to the therapeutic treatment of impotentia coeundi and provides a new preparation for topical use which is active in the said therapeutic treatment.

The use of vasoactive agents such as papaverine and phentolamine in the diagnosis of impotentia coeundi is already known. Such agents have already been used in the cavernous pharmacoinduction method (CPI) in the Virag test exclusively for diagnostic purposes.

It has now been found that, surprisingly, these agents can also be used for therapeutic purposes in preparations for topical use.

Hence a subject of the present invention is a preparation including papaverine and phentolamine for topical use in the therapeutic treatment of impotentia coeundi.

A further subject of the invention is constituted by the use of papaverine and phentolamine in the preparation of a composition for topical use which is useful in the said therapeutic treatment.

The preparation according to the invention is preferably in the form of a gel and includes papaverine at a concentration of from 0.15 to 3.3% by weight, preferably from 0.15 to 2%, and phentolamine at a concentration of no more than 0.3% by weight, preferably from 0.015 to 0.2% with respect to the weight of the composition.

Any gel which does not give rise to side effects may be used as the vehicle in the preparation; for example starch glyceroate may be used to advantage, together with the usual preservatives and stabilisers.

According to a further aspect, the preparation may include nitroglycerine as a further active agent for topical use in a concentration of between 0.01 and 1% by weight of the weight of the preparation.

The therapeutic effectiveness of the preparation according to the invention has been demonstrated by means of a series of clinical tests carried out on a significant number of patients.

188 patients who had been suffering from impotentia coeundi for periods which varied from a few months to several years were considered over a period of twelve months. After accurate case-history, clinical and metabolic screening, the patients underwent an induced erection test with 20 mg of papaverine and 2 mg of phentolamine. Truly organic cases of impotence thus having been identified (not more than 10% of those presented for observation), CPI cycles were planned in all cases with the same composition and dosage as used at the diagnostic stage. Sixty patients, selected from the most homogeneous in age, social standing and lifestyle, without diabetes, hypertension or ulcers, were divided into three subgroups.

The first group underwent CPI treatment only; the second group underwent treatment with CPI and a placebo constituted by a gel base and the third group underwent treatment with CPI plus a preparation according to the invention in the form of a gel having the following composition:
papaverine 2% by weight
phentolamine 0.1% by weight in a water-soluble gel.

After one year of observation, the nature of the preparation for topical use actually used having been kept secret, the following conclusions were reached:
- of all the patients, only 10% did not respond to treatment.

In these cases the Virag test was still negative;
- of the remaining 90%, the large majority (85%) responsed satisfactorily to the Virag test which they underwent and the consequent therapeutic CPI cycle associated with, and then replaced by, the simple application of the vasoactive gel, resolved the case extremely well. In the remaining 15% of the second category, while there was no response to the pharmacoerection test in the course of the first session and there was therefore an absence of any bio-feedback, and indeed reinforcement of the negative psychogenic factors, there were subsequent episodes of erection and intercourse. The treatment was again carried out satisfactorily, in these cases with the ambulatory topical use of the vasoactive gel, possibly with additional, sporadic CPI support.

After many months, the cases under consideration did not have any significant relapse.

In the final subgroup, there was a quicker and more complete erection response which was statistically significant. The use of the active gel enabled the dose of papaverine and phentoalamine injected to be reduced both at the diagnostic stage and in the therapeutic stage, enabled shorter therapeutic CIP cycles to be used successfully and enabled better results to be obtained in terms of greater frequency and quality of intercourse than in the cases treated solely with CPI or CPI plus placebo, the latter being practically identical.

The clinical tests described above therefore show the therapeutic effectiveness of the preparation according to the invention both alone and in combination with an interacavernous pharmacoinduction cycle, whereby the preparation can be used to advantage by sufferers for treatment at home.

The result is even more surprising since the effectiveness of the active principles, papaverine and phentolamine, or of vaso-dilatory drugs in topical application to the skin, is not in fact predictable or expected.

In view of the improvements achieved in terms of improved quality of intercourse, the topical use of the active agents papaverine and phentolamine, possibly in combination with nitroglycerine, as aids to sexual performance can also be envisaged; the use of prophylactic devices containing these agents in solution or in a water-soluble gel can thus be envisaged.

It has been further ascertained that the preparation of the invention may be used also for the therapeutical treatment of frigidity in women. It is therefor understood that the term "impotentia coeundi" as used herein includes as well female frigidity.

## Claims

1. A preparation including an effective quantity of papaverine and phentolamine in a vehicle for topical use in the therapeutic treatment of impotentia coeundi.

2. A preparation according to Claim 1, in the form of a gel including papaverine at a concentration of from 0.15 to 3.3% by weight and phentolamine at a concentration no greater than 0.3% by weight of the weight of the preparation.

3. A preparation according to Claim 1 or Claim 2, in which papaverine is present at a concentration of from 0.15 to 2% by weight and phentolamine is present at a concentration of from 0.015 to 2% by weight of the composition.

4. A preparation according to Claim 1, Claim 2 or Claim 3, further including a nitroglycerine base for topical use at a concentration, of between 0.01% and 1% by weight of the weight of the preparation.

5. The use of papaverine and phentolamine for the preparation of a gel composition for topical use, useful in the therapeutic treatment of impotentia coeundi.

6. A prophylactic device containing papaverine and phentolamine.

7. A prophylactic device according to Claim 6 further including a nitroglycerine base for topical use.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | BIOLOGICAL ABSTRACTS, vol. 84, no. 12, 1987, abstract no. 122678, Biological Abstracts, Inc., Philadelphia, PA, US; G. WILLIAMS et al.: "Impotence: Treatment by autoinjection of vasoactive drugs", & BR MED J 295(6598), 595-596, 1987 * Abstract * | 1 | A 61 K 31/485 A 61 K 9/06 // (A 61 K 31/485 A 61 K 31:415 A 61 K 31:21 ) |
| X | BIOLOGICAL ABSTRACTS, vol. 83, no. 10, 1987, abstract no. 100737, Biological Abstracts, Inc., Philadelphia, PA, US; M.A. ROBINETTE et al.: "Intracorporal injection of papaverine and phentolamine in the management of impotence", & BR J UROL 58(6): 692-695, 1986 * Abstract * | 1-3 | |
| A | CHEMICAL ABSTRACTS, vol. 76, 1972, page 34, abstract no. 148919w, Columbus, Ohio, US; I.E. KISIN et al.: "Autoregulatory vasoconstriction and vasodilator drugs", & ARCH. INT. PHARMACODYN. THER. 1972, 195(1), 24-32 * Abstract * | 1,4 | TECHNICAL FIELDS SEARCHED (Int. Cl.4) A 61 K |

The present search report has been drawn up for all claims

| Place of search | Date of completion of the search | Examiner |
|---|---|---|
| THE HAGUE | 26-09-1989 | PEETERS J.C. |